# EUROPEAN PATENT SPECIFICATION

(11) **EP 1 520 568 B1**
(45) Date of publication and mention of the grant of the patent: **27.07.2011**
(21) Application number: 03425646.1
(22) Date of filing: 03.10.2003
(51) Int. Cl.: A61F 7/00

(54) **Thermal exchange apparatus, particularly for the application of thermal treatments**
Wärmeaustauschvorrichtung, insbesondere zur Anwendung der Wärmebehandlung
Appareil d'échange thermique, en particulier pour l'application du traitement thermique

(43) Date of publication of application: 06.04.2005
(73) Proprietor: Zanotti, Mario, 46029 Suzzara (Mantova) (IT)
(72) Inventor: Zanotti, Mario, 46029 Suzzara (Mantova) (IT)
(74) Representative: Pulieri, Gianluca Antonio

(56) References cited:
- EP-A1- 0 463 837
- FR-A3- 2 696 342
- US-A- 2 726 658
- US-A- 3 504 674
- US-A- 3 888 259
- US-A- 3 916 911
- US-A- 4 184 537
- US-A1- 2003 060 864

## Description

The present invention refers to a thermal exchange apparatus, which has preferred although not exclusive use as apparatus for localised application of thermal treatments to body parts of living beings, particularly animals or people, even more particularly to horses.

In the present description and the subsequent claims, under the expression localised application of thermal treatments, the application, according to predetermined criteria, of heat or cold, or else even of a suitable combination of alternating heat or cold, to parts of a human or animal body, in particular, but not exclusively, for therapeutic use, is meant.

In the veterinary and medical field the beneficial effects of thermal therapy in the treatment of various types of traumas or pathologies are known, both to alleviate pain and as adjuvant for the healing process. Besides for strictly therapeutic purposes, the application of thermal treatments to human or animal body parts is also useful, in particular in the sport field, to tone up muscle or joints in anticipation of or after prolonged or intense physical effort, in order to avoid harmful contractures.

The thermal treatments cited above are particularly appreciated for horses, above all for treating and preventing pathologies involving limbs, such as, for example, haematomas, sprains, torn muscles; they are also validly used to relieve and tone up the limbs of horses which must bear prolonged or intense stresses in athletic competitions.

Different systems and devices for the application of thermal treatments to animal or human body parts, and, in particular, horse body parts, are known in the art.

For the application of cold, ice is commonly used. This system is easy and quick to use, but has the drawback of being able to keep the desired cold for a limited time, making frequent replacements necessary if the duration of the treatment goes on for a long time. Moreover, there is the need to have freezer units, not always available for outdoor applications, like, for example, in horse stables.

Bags containing coolants or substances which perform a refrigerating action as a consequence of endothermic chemical reactions are also used; the use of such bags allows the cold to be maintained for a longer time, but has a series of drawbacks. Bags containing coolants can be used many times over, however their use is laborious due to the need of having freezing units to cool down the bags with the coolant and keep them at a low temperature until the time of application. Bags containing substances which perform a refrigerating action as a consequence of endothermic chemical reactions are easier to use, with respect to the previous ones, but can each be used just once, because such substances lose their refrigerating capability after the first use. Both such substances and the coolants cited above are, however, more expensive and more difficult to obtain with respect to ice.

Drawbacks common to all of the cited systems or devices are also the difficulty of application to the part to be treated, because of the lack of conformability due to rigidity, particularly in the case of ice or bags containing coolants, and the impossibility of precise control of the temperature. In particular, moreover, there is the fact that the temperature can be too cold for the required application.

For the application of heat analogous devices are known, such as bags containing hot water, which have some of the drawbacks mentioned in relation to the systems and devices for the application of cold, particularly the ability to maintain the heat only for a limited time and the impossibility of precise control of the temperature.

A hypothermia device is also known from US 2726658.

Again for the application of heat, electrical resistance devices are also used, which make use of the heat generated by one or more resistances flown by electric current. Such devices allow a more accurate temperature control and allow also long treatments, but require the availability of a power supply, and that devices flown by electric current are brought into direct contact with the body, with potential safety problems.

In the equestrian field, devices distributed by BADI FARM S.r.l. (Albusciago di Sumirago (VA) - Italy) under the trade name WATER FLOW© for thermal treatments and hydromassage of horse limbs are known, comprising microporous fabric bags to be applied to the limbs, which are connected by means of ducts to a waterworks; the water flows through the aforementioned ducts to the bags applied to the zones to be treated and flows out from the micropores, simultaneously carrying out a thermal action (cold or heat according to the temperature of the water) and a hydromassage action. Such a device, although simple and cost-effective, necessarily requires connection to a waterworks or another water source, and in any case has the aforementioned drawback of reduced possibility of fine adjustment of the temperature of the treatment; moreover, it is not always desirable for the part to be treated to be constantly wetted by water that flows out from the micropores.

The technical problem at the basis of the present invention is therefore to provide a thermal exchange apparatus, particularly for the application of localised thermal treatments, which overcomes the drawbacks cited above with reference to the prior art, in particular as far as the flexibility and ease of use are concerned.

The invention is defined in claim 1. It relates to a thermal exchange apparatus comprising a primary circuit for circulating a primary thermal carrier fluid, said primary circuit being enclosed in a main body and comprising at least one compressor, means for imposing a temperature change on said primary thermal carrier fluid, an expansion member for said primary thermal carrier fluid and a first heat exchanger, at which said primary thermal carrier fluid undergoes a temperature change opposite in sign, characterised in that a secondary circuit for circulating a secondary thermal carrier fluid is thermally coupled with said primary circuit at said first heat exchanger, said secondary circuit comprising at least one second heat exchanger between said secondary thermal carrier fluid and an external body, said second heat exchanger being external to said main body.

In such a way, whilst the primary circuit embodies a thermal cycle for producing cold or heat, thanks to the use of said secondary circuit and of said at least one second heat exchanger it is possible to easily transfer the cold and/or heat to the outside of said main body of the apparatus and apply it to an external body, even at a distance from such a main body. In such a way a substantial flexibility and ease of use of the apparatus is overall attained.

According to a preferred embodiment of the invention, said at least one second heat exchanger comprises a bag made of flexible material, flowable by said secondary thermal carrier fluid. This advantageously allows providing an exchanger adaptable to the part to be thermally treated, so as to ensure a correct and comfortable application thereof.

Said flexible material is preferably polyvinyl chloride (PVC), a material having an advantageous balance between strength and flexibility.

Advantageously, from the point of view of cost-effectiveness and simplicity of manufacture, said bag comprises a zig-zagging path obtained through heat-sealing of portions of the walls of said bag. Such a zig-zagging path for the secondary thermal carrier fluid allows the thermal exchange between such a fluid and said external body to be improved, for a better and more uniform application of the thermal treatment.

According to a preferred embodiment, a support sack having means for removably fastening to said external body is removably associated with said bag. Advantageously, therefore, said bag can be produced according to a standard size and shape, whereas said support sack can have different shapes to better adapt to said external body or parts thereof; it is thus possible to associate the same bag each time with different support sacks according to the application. The use of a removable support sack also allows greater hygiene of use.

Preferably, said support sack is made of flexible material.

Preferably moreover, said removable fastening means is adjustable.

Both of the provisions allow better adaptability to said external body, or parts thereof, of said support sack, which can also thus be produced according to standard shapes and sizes and subsequently adapted at the time of application. Examples of preferred removable fastening means comprise hooks and loops systems (Velcro™), buckles or strings, possibly elastic.

Preferably moreover, said support sack comprises a thermally insulating main surface; such a thermally insulating surface advantageously ensures the thermal insulation of said bag towards the external environment when inserted in said support sack for a thermal treatment, so as to increase the overall thermal efficiency of the apparatus.

Preferably, said support sack also comprises a main surface made of anti-allergenic material; the presence of such a surface is particularly advantageous when the thermal treatment is to be applied to an animal or human body.

In a preferred embodiment of the invention, said at least one second heat exchanger is connected to said main body through flexible tubular ducts; this aspect advantageously allows said at least one second exchanger to be freely moved to bring it at said external body, possibly even at a distance from the main body of the thermal exchange apparatus. This gives further flexibility and ease of use to the apparatus as a whole, allowing in particular a certain freedom of movement for a living being receiving the thermal treatment.

Preferably, said flexible tubular ducts are thermally insulated, so as to be able to keep said secondary thermal carrier fluid at the temperature required by the thermal treatment, with a consequent improvement of the overall efficiency of the apparatus.

Preferably moreover, said tubular ducts are removably connected to said main body and/or to said at least one second heat exchanger. In this way it is advantageously possible to replace said ducts and/or said bags in the case of wear or failure, and a flexibility in the number of bags used in different thermal treatments is also achieved.

According to a preferred embodiment of the invention, said primary circuit comprises means for switching between a first operating mode for producing cold at said external body and a second operating mode for producing heat at said external body; this feature offers the notable advantage of allowing the same thermal exchange apparatus to be used to administer cold or heat separately, and/or heat and cold alternately.

Preferably, said switching means comprises a four-way valve; advantageously, such a valve embodies a simple device for modifying the path of said primary thermal carrier fluid in said primary circuit and reversing the thermal cycle. Through such a valve it is, indeed, possible to switch between operation with a thermal cycle for producing cold and operation with a thermal cycle for producing heat.

In a preferred embodiment, said means for imposing a temperature change on said primary thermal carrier fluid comprises an air heat exchanger; advantageously, said heat exchanger allows the air of the external environment, which is always available, to be exploited as a heat source or well, respectively in the first or in the second operating mode defined above, to change the temperature of said primary thermal carrier fluid.

Said secondary circuit can further comprise a selectively actuable electroheating element. Said selectively actuable electroheating element can advantageously be used to supplement the heat supplied to said secondary fluid from the primary thermal carrier fluid in said second operating mode (production of heat).

Said expansion member of said primary circuit can be a capillary or else a thermostat valve. Both devices are suitable for operating in said first and second operating modes. A capillary has the advantage of being a particularly simple device, without moving members and therefore with reduced chances of failure and wear; a thermostat valve can, on the other hand, be advantageous in the case where higher thermal powers have to be processed.

Said primary thermal carrier fluid is a coolant, for example Freon R134A and/or R404A. Although it could be a coolant too, the secondary thermal carrier fluid is instead preferably water, which has the advantage of being commonly available at virtually zero cost and of not being a dangerous or harmful fluid. Its use in the secondary circuit is satisfactory, considering in particular the moderate performance required in the operating mode for producing cold.

Said secondary circuit preferably comprises a pump for forcedly circulating said secondary thermal carrier fluid; this advantageously allows said secondary thermal carrier fluid to be circulated without restrictions on the configuration or length of said secondary circuit.

The thermal exchange apparatus according to the present invention further comprises means for controlling and adjusting the thermal exchange between said secondary thermal carrier fluid and said external body, in order to advantageously be able to precisely control and adjust the parameters, in particular duration and temperature, under which said heat exchange is accomplished.

Said control and adjustment means preferably comprises a timer, a mode selector and/or a programmable electronic control unit, which allow the duration and/or the type (cold, heat and/or alternating cold and heat, in all cases with predeterminable temperatures) of the thermal treatments to be set, through setting by the user and/or through preset cycles.

The thermal exchange apparatus according to the present invention is preferably supplied with alternating current, to be able to be connected to a common electrical mains.

As an alternative or in addition, it can also comprise a power supply battery; this feature advantageously allows said apparatus to be made autonomous as far as the electric power supply is concerned, making its portability easier.

Preferably, the main body of the thermal exchange apparatus of the invention is portable. The apparatus is therefore not forced to remain in a particular environment, but can be moved according to the need, even outdoors, with a further advantageous flexibility of use.

Further characteristics and advantages of the present invention.shall become clearer from the following detailed description of a preferred embodiment thereof, made with reference to the attached drawings. In such drawings:
- figure 1 is a perspective view of a thermal exchange apparatus, particularly for the application of localised thermal treatments, according to a preferred embodiment of the present invention;
- figure 2 is a perspective view of a preferred embodiment of a second heat exchanger of the thermal exchange apparatus of figure 1;
- figure 3 is an exploded perspective view of the main body of the thermal exchange apparatus of figure 1, where electrical connections and fluid connections are omitted, and
- figures 4 and 5 are block diagrams of the thermal exchange apparatus of figure 1, relative to a first and a second operating mode thereof, respectively.

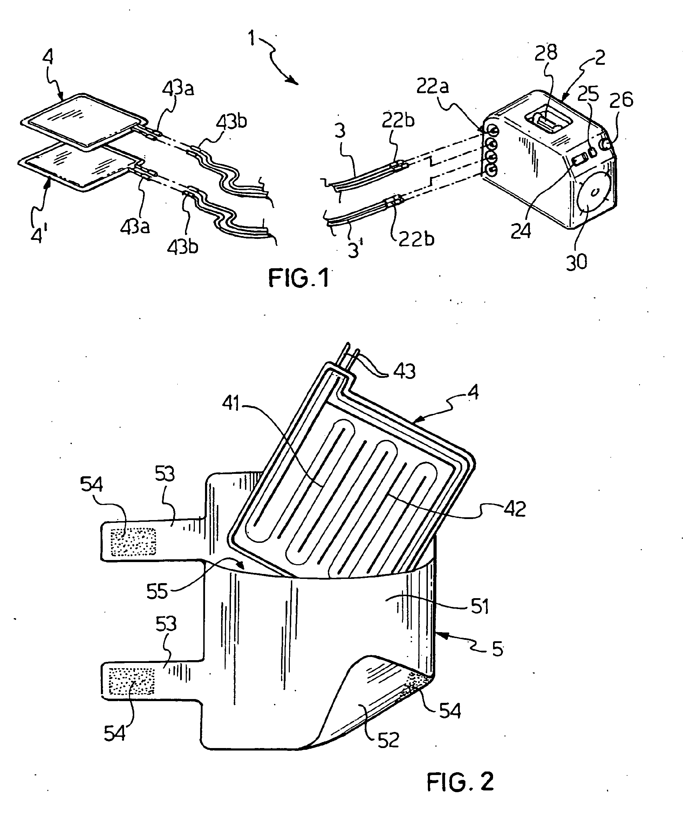

With reference to figure 1, a thermal exchange apparatus 1 essentially comprises: a main body 2, two bags 4, 4' for the application of thermal treatments to an external body (not shown in the figures) and two pairs of flexible and thermally insulated tubular ducts 3, 3'. The pairs of ducts 3, 3' connect the bags 4, 4' with the main body 2, ensuring that they are connected to a secondary circuit for circulating a secondary thermal carrier fluid (as illustrated in greater detail hereafter) having a given temperature, defined according to the type of thermal treatment to be applied on said external body.

The bags 4, 4' are made of flexible material, preferably polyvinyl chloride (PVC), to be easily adapted to the configuration of the external body to which the thermal treatment is intended to be applied, or to parts thereof. Inside such bags 4, 4' a zig-zagging path 41 is obtained, through a plurality of suitable separating partitions 42, which can be formed, for example, through heat-sealing of portions of the walls of such bags. The zig-zagging path 41 terminates at inlet and outlet fittings 43a of the secondary thermal carrier fluid, suitable for connecting with a respective connector 43b, provided at the end of the ducts 3, 3'.

Alternatively, the zig-zagging path 41 can be obtained by suitably deforming a flexible tubular duct, which can then be fixed inside the bags 4, 4' so as to keep the shape given to it and to be easier to apply. It is also possible to make bags 4, 4' without any path inside, with the advantage of greater simplicity of manufacture and, consequently, of greater cost-effectiveness, without departing from the scope of protection of the present invention.

At the time of use, the bags 4, 4' are removably associated each with a support sack 5, as shown in figure 2. Such a support sack, also made of flexible material, comprises a main surface 51 and an opposite main surface 52, between which a pocket 55 is defined, suitable to receive a respective bag 4, 4'. The sack 5 further comprises means 54 to allow it to be removably fixed to the external body for which it is intended, or to part thereof. Such means, in the illustrated embodiment, comprises a pair of elements of a hooks and loops system (Velcro™) on a first surface of closing wings 53 and a corresponding pair of such elements on the opposite main surface 52 of the sack 5 (of which just one is visible in figure 2). Alternatively or in addition, the removable fastening means 54 may comprise a plurality of buckles or strings, possibly elastic.

The main surface 51 is made of anti-allergenic material, whereas the opposite main surface 52 is thermally insulating. When the support sack 5 with the respective bag 4, 4' is applied to a body, in particular a human or animal body, the main surface 51 made of anti-allergenic material is brought into contact with the body, whereas the opposite thermally insulating main surface 52 is left outwards, so that the fluid circulating in the bag 4, 4' preferentially exchanges heat with the body, while remaining thermally insulated with respect to the rest of the external environment.

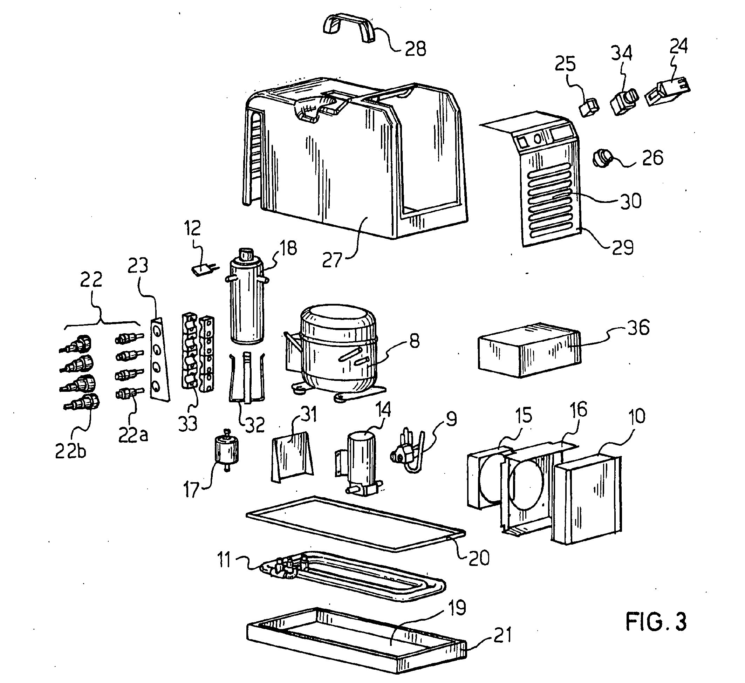

With reference to figures 3, 4 and 5, the main body 2 of the thermal exchange apparatus 1 houses within it a plurality of devices forming part of the primary circuit 6, mounted on a support base 20, in particular: a compressor 8; a four-way valve 9 electrically controlled by means of a solenoid (not shown); an air exchanger 10, with a fan 15 and a collector 16 associated; a filter 17, having the function of holding impurities and possible humidity present inside the primary circuit, and a first heat exchanger 11 at which thermal exchange takes place between a primary thermal carrier fluid and the secondary thermal carrier fluid. According to a preferred embodiment, the heat exchanger 11 is a concentric tube exchanger and comprises an outer tubular duct and an inner tubular duct.

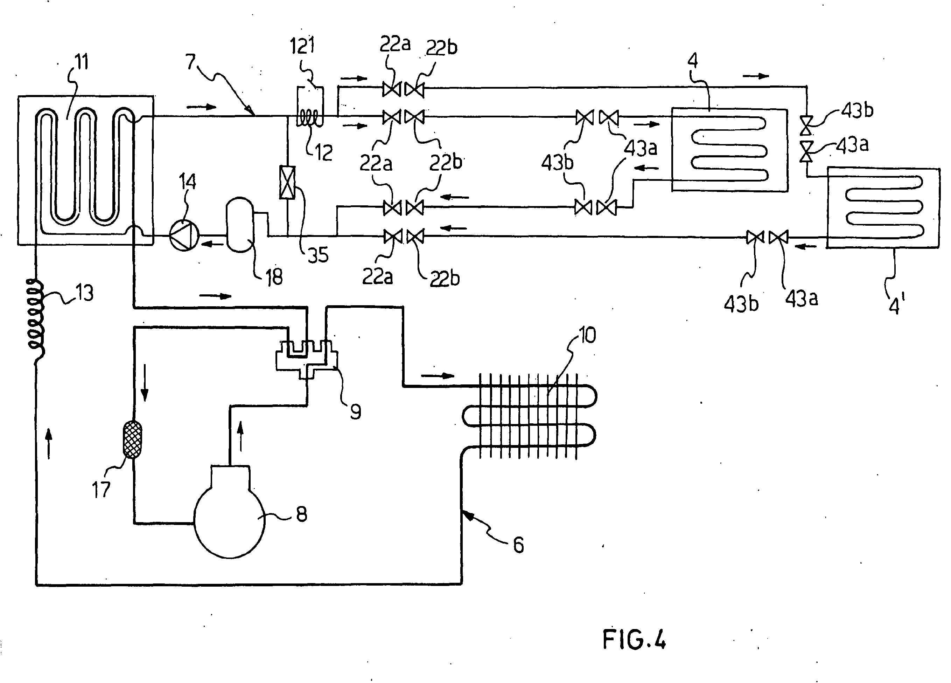

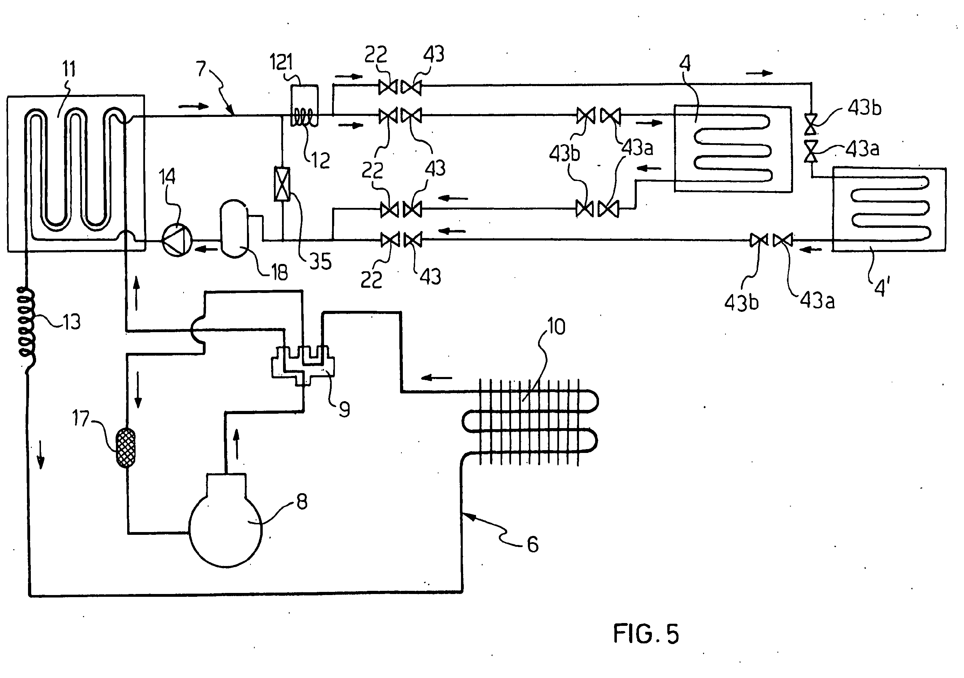

The primary thermal carrier fluid flows in the inner tube, whereas the secondary thermal carrier fluid flows in the gap between inner tube and outer tube. The heat exchanger 11 is shaped so as to be able to be housed in a volume 19, defined between the support base 20 and a base 21 of the main body 2. The bases 20, 21 are internally insulated, so as to thermally insulate the heat exchanger 11 with respect to the external environment. The primary circuit 6 further comprises an expansion member 13 (shown for clarity only in the block diagrams of figure 4 and 5), which is typically a capillary. Alternatively, in the case where high thermal powers are present, the use of a thermostat valve can be foreseen.

The main body 2 also houses some of the devices of the secondary circuit 7, in particular, besides the first heat exchanger 11 just described above and shared with the primary circuit 6: a pump 14, with a relative support 31, for circulating the secondary thermal carrier fluid; a storage tank 18 for the secondary thermal carrier fluid with relative support 32; a selectively actuable electroheating element 12; two pairs of rapid fastening/releasing connectors 22, with relative male 22a and female 22b elements, for example of the bayonet type, for the connection of the flexible tubular ducts 3, 3' with the main body 2, and a collar 33, with relative supporting upright 23, for mounting the attachments 22.

Inside main body 2 there are also electric power supply devices, only schematically illustrated under reference numeral 36 in figure 3, including a power supply transformer to convert mains alternating current into low direct supply voltage, for example 12V.

In addition or as an alternative to mains power supply, it is possible to foresee the use of a battery (not shown in the figures), preferably rechargeable, to make the thermal exchange apparatus 1 not reliant upon an external power supply.

Adjustment and control devices, comprising a programmable electronic control unit 24, an operating mode (cold/heat) selector 34 and a timer 26, and a general switch 25 are housed in the main body 2 and accessible from the outside.

All of the devices just mentioned above are enclosed in a casing 27, which is removably associated with the base 21. Such a casing 27 is equipped with grip means 28 for transporting the main body 2, and with a removable front panel 29 equipped with an aeration grid 29 for the air exchanger 10, and with slots for the access to the aforementioned adjustment and control devices 24-26, 34.

For the sake of clarity, in the exploded view of figure 3 the electrical circuitry and the connection tubing between the components described above have not been illustrated.

The fluid connection of the aforementioned components and the operation of the thermal exchange apparatus 1 are described with reference to the block diagrams of figure 4, which illustrates a first operating mode thereof for the production of cold at an external body (not shown), and of figure 5, which illustrates a second operating mode thereof for the production of heat at said external body. In such figures, the primary circuit 6 and the secondary circuit 7 and the direction of circulation, highlighted by the arrows, of the primary thermal carrier fluid, typically Freon R134A and/or R404A, and of the secondary thermal carrier fluid, typically water, are schematically shown.

In the first operating mode (figure 4), the four-way valve 9 is in a first operating configuration, through which the delivery of the compressor 8 is connected with the air exchanger 10 and its suction with the first heat exchanger 11, through the filter 17. In the air exchanger 10 the primary thermal carrier fluid, which has previously been compressed by the compressor 8, undergoes a temperature decrease, transferring a first amount of heat to the external environment.

The primary thermal carrier fluid then reaches the expansion capillary 13, within which it expands, and then the first heat exchanger 11, at which it undergoes a temperature increase, absorbing a second amount of heat from the secondary thermal carrier fluid. The primary thermal carrier fluid finally returns, through the four-way valve 9 and the filter 17, to the suction of the compressor 8.

The secondary thermal carrier fluid is circulated in the secondary circuit 7 through the circulation pump 14. It decreases its temperature at the first heat exchanger 11, transferring the aforementioned second amount of heat to the primary thermal carrier fluid, and therefore cooling down, and then reaches at the bags 4, 4', having the function of second heat exchangers, at the desired temperature (cold) for the thermal treatment on the external body, on which such bags have previously been applied.

Whilst it flows in the zig-zagging paths 41 of the bags 4, 4' (figure 2), the secondary thermal carrier fluid takes away heat from the external body, cooling it down. The secondary thermal carrier fluid finally returns, after having passed through the tank 18 and the pump 14, to the first heat exchanger 11 to transfer the heat absorbed from the external body to the primary thermal carrier fluid, once again decreasing its own temperature.

The electroheating element 12 is not active, as schematically represented by the switch 121 in the open position.

A by-pass valve 35 (shown only in the block diagrams of figure 4 and 5) is also preferably provided so as to allow sending part of the flow rate of secondary thermal carrier fluid directly to the tank 18 without making it flow through the bags 4, 4', in the case where it detects overpressures in the secondary circuit, in order to protect the bags 4, 4' from such overpressures which could damage them or, in the worst case, make them burst.

In the second operating mode (figure 5), the four-way valve 9 is in a second operating configuration, through which the suction of the compressor 8 is connected with the air exchanger 10, through the filter 17, and its delivery is connected with the first heat exchanger 11. In the air exchanger 10 the primary thermal carrier fluid undergoes a temperature increase, receiving a first amount of heat from the external environment. After having been heated, the primary thermal carrier fluid, through the four-way valve 9 and the filter 17, reaches the compressor 8, where it is compressed, and then, again through the four-way valve 9, the first heat exchanger 11, at which it undergoes a temperature decrease, transferring a second amount of heat to the secondary thermal carrier fluid. The primary thermal carrier fluid finally returns, after having expanded through the expansion capillary 13, to the air exchanger 10.

The secondary thermal carrier fluid, circulated in the secondary circuit 7 through the circulation pump 14, increases its temperature at the first heat exchanger 11, receiving the aforementioned second amount of heat from the primary thermal carrier fluid. Such a second amount of heat can also be increased (for example, when the temperature of the external environment is too low, as it could happen in the winter months) through the heating obtained through the actuation of the electroheating element 12. This condition is schematically represented in figure 5 by the switch 121 of the power supply circuit in closed position.

The secondary thermal carrier fluid then reaches the bags 4, 4', at the desired temperature (hot) for the thermal treatment on the external body, on which such bags have previously been applied.

Whilst it flows in the zig-zagging paths 41 (figure 2), the secondary thermal carrier fluid transfers heat to the external body, heating it up, and undergoing a temperature decrease. The secondary thermal carrier fluid finally returns, after having passed through the tank 18 and the pump 14, to the first heat exchanger 11 to receive from the primary thermal carrier fluid the heat previously transferred to the external body and once again increase its own temperature.

The switching between the two operating modes, based upon the type of thermal treatment to be applied, can be carried out manually by a user, possibly with the aid of the operating mode (cold/heat) selector 34 and the timer 26, or else can take place automatically, under the control of the programmable electronic control unit 24. Such a programmable electronic control unit 24, in carrying out the control, operatively interacts with devices for controlling the temperature of the primary thermal carrier fluid (not shown in the figures), in particular a thermostat and temperature sensors, and with actuators (not shown in the figures) acting upon the devices of the primary circuit 6 and secondary circuit 7, in particular on the four-way valve 9 and on the power supply circuit for the electroheater 12.

The thermal exchange apparatus 1 has a preferred use for the application of localised thermal treatments to parts of the body of living beings. Such localised thermal treatments are preferably therapeutic'treatments.

The thermal exchange apparatus 1 is used for the application of thermal treatments to horses, in particular for the treatment or prevention of pathologies or fatigue of the limbs. It is therefore advantageous to have an apparatus equipped with a plurality of second heat exchangers, such as bags 4, 4', for carrying out a thermal treatment on many limbs simultaneously. The present detailed description has been made with reference to an embodiment comprising two second heat exchangers 4, 4' for the application of a thermal treatment, but it is manifest that an embodiment comprising many second heat exchangers (for example four, to treat the four legs of a horse simultaneously), or else even a single second heat exchanger, fully falls within the scope of the present invention.

Further variations of the preferred embodiment described above, which shall not be described here in detail, but in any case fall within the scope of the invention, comprise, by way of an example only: the elimination of the four-way valve 9, should the thermal exchange apparatus be intended to operate just for the production of cold or just for the production of heat; the possibility of including, among the means for imposing a temperature change on the primary thermal carrier fluid, a selectively actuable electroheating element; the use of bags 4, 4' equipped with fastening means themselves, so as to avoid the further use of support sacks 5.

It shall also be understood that some elements can be missing, in particular the filter 17, the tank 18, and, as mentioned, the electroheating element 12.

It shall also be clear that the bag(s) 4, 4' can be unremovably fixed in the secondary circuit 7.

## Claims

1. Thermal exchange apparatus (1) comprising;
- a primary circuit (6) for circulating a primary thermal carrier fluid, said primary circuit being enclosed in a main body (2) and comprising at least one compressor (8), means (10) for imposing a temperature change on said primary thermal carrier fluid, an expansion member (13) for said primary thermal carrier fluid and a first heat exchanger (11), at which said primary thermal carrier fluid undergoes a temperature change opposite in sign, and
- a closed secondary circuit (7) for circulating a secondary thermal carrier fluid that is thermally coupled with said primary circuit at said first heat exchanger (11), said secondary circuit comprising:
a) at least one second heat exchanger (4, 4') between said secondary thermal carrier fluid and an external body, said second heat exchanger (4, 4') being external to said main body (2); and
b) a storage tank (18) for the secondary thermal carrier fluid;
**characterized in that** said secondary circuit (7) further comprises a by-pass valve (35) which, in case where it dectects overpressures in the secondary circuit, is adapted to allow sending part of the flow rate of the secondary thermal carrier fluid to the storage tank (18) without making it flow through the second heat exchanger (4, 4').

2. Thermal exchange apparatus (1) according to claim 1, wherein said at least one second heat exchanger comprises a bag (4, 4') made of flexible material, said bag being flowable (41) by said secondary thermal carrier fluid.

3. Thermal exchange apparatus (1) according to claim 2, wherein said flexible material is polyvinyl chloride.

4. Thermal exchange apparatus (1) according to claim 2 or 3, wherein said bag (4, 4') comprises a zig-zagging path (41) between heat-sealed portions (42) of the walls of said bag (4, 4').

5. Thermal exchange apparatus (1) according to any one of claims 2 to 4, wherein a support sack (5) having means (54) for removably fastening to said external body is removably associated with said bag (4, 4').

6. Thermal exchange apparatus (1) according to claim 5, wherein said support sack (5) is made of flexible material.

7. Thermal exchange apparatus (1) according to claim 5 or 6, wherein said removable fastening means (54) is adjustable.

8. Thermal exchange apparatus (1) according to any one of claims 5 to 7, wherein said support sack (5) comprises a thermally insulating main surface (52).

9. Thermal exchange apparatus (1) according to any one of claims 5 to 8, wherein said support sack (5) comprises a main surface (51) made of anti-allergenic material.

10. Thermal exchange apparatus (1) according to any one of the previous claims, wherein said at least one second heat exchanger (4, 4') is connected to said main body (2) through flexible tubular ducts (3, 3').

11. Thermal exchange apparatus (1) according to claim 10, wherein said flexible tubular ducts (3, 3') are thermally insulated.

12. Thermal exchange apparatus (1) according to claim 10, wherein said flexible tubular ducts (3, 3') are removably connected to said main body (2) and/or to said at least one second heat exchanger (4, 4').

13. Thermal exchange apparatus (1) according to any one of the previous claims, wherein said primary circuit (6) further comprises means (9) for switching between a first operating mode for producing cold at said external body and a second operating mode for producing heat at said external body.

14. Thermal exchange apparatus (1) according to claim 13, wherein said switching means comprises a four-way valve (9).

15. Thermal exchange apparatus (1) according to any one of the previous claims, wherein said means (10) for imposing a temperature change on said primary thermal carrier fluid comprises an air heat exchanger (10).

16. Thermal exchange apparatus (1) according to any one of the previous claims, wherein said secondary circuit (7) further comprises a selectively actuable electroheating element (12).

17. Thermal exchange apparatus (1) according to any one of the previous claims, wherein said expansion member (13) of said primary circuit is selected from a capillary and a thermostat valve.

18. Thermal exchange apparatus (1) according to any one of the previous claims, wherein said primary thermal carrier fluid and said secondary thermal carrier fluid are a coolant and water, respectively.

19. Thermal exchange apparatus (1) according to any one of the previous claims, wherein said secondary circuit (7) comprises a pump (14) for forcedly circulating said secondary thermal carrier fluid.

20. Thermal exchange apparatus (1) according to any one of the previous claims, further comprising means (24-26, 34) for controlling and adjusting the heat exchange between said secondary thermal carrier fluid and said external body.

21. Thermal exchange apparatus (1) according to claim 20, wherein said control and adjustment means (24-26, 34) comprises a timer (26), a mode selector (34) and/or a programmable electronic control unit (24).

22. Thermal exchange apparatus (1) according to any one of the previous claims, supplied with alternating current.

23. Thermal exchange apparatus according to any one of the previous claims, comprising a power supply battery.

24. Thermal exchange apparatus (1) according to any one of the previous claims, wherein said main body (2) is portable.

## Patentansprüche

1. Wärmeaustauschvorrichtung (1), umfassend:
- eine primäre Schaltung (6) zum Umwälzen eines primären Wärmeträgerfluides, wobei die primäre Schaltung in einem Hauptkörper (2) eingeschlossen ist und wenigstens einen Kompressor (8), Mittel (10) zum Bewirken einer Temperaturänderung an dem primären Wärmeträgerfluid, ein Expansionsglied (13) für das primäre Wärmeträgerfluid und einen ersten Wärmetauscher (11), an dem das primäre Wärmeträgerfluid eine dem Vorzeichen nach entgegengesetzte Temperaturänderung erfährt, umfasst und
- eine geschlossene sekundäre Schaltung (7) zum Umwälzen eines sekundären Wärmeträgerfluides, das thermisch mit der primären Schaltung an dem ersten Wärmetauscher (11) gekoppelt ist, wobei die sekundäre Schaltung umfasst:
a) wenigstens einen zweiten Wärmetauscher (4, 4') zwischen dem sekundären Wärmeträgerfluid und einem äußeren Körper, wobei der zweite Wärmetauscher (4, 4') außerhalb des Hauptkörpers (2) befindlich ist; und
b) einen Speichertank (18) für das sekundäre Wärmeträgerfluid;
**dadurch gekennzeichnet, dass** die sekundäre Schaltung (7) des Weiteren ein Bypassventil (35) umfasst, das für den Fall, dass es Überdrücke in der sekundären Schaltung erfasst, dafür ausgelegt ist zu ermöglichen, einen Teil der Flussrate des sekundären Wärmeträgerfluides an den Speichertank (18) zu senden, ohne dass es zuließe, dass dieses durch den zweiten Wärmetauscher (4, 4') fließen würde.

2. Wärmeaustauschvorrichtung (1) nach Anspruch 1, wobei der wenigstens eine zweite Wärmetauscher einen Beutel (4, 4') umfasst, der aus einem flexiblen Material besteht, wobei der Beutel von dem sekundären Wärmeträgerfluid durchfließbar (41) ist.

3. Wärmeaustauschvorrichtung (1) nach Anspruch 2, wobei das flexible Material Polyvinyl-chlorid ist.

4. Wärmeaustauschvorrichtung (1) nach Anspruch 2 oder 3, wobei der Beutel (4, 4') einen Zickzackweg (41) zwischen wärmeabgedichteten Abschnitten (42) der Wände des Beutels (4, 4') umfasst.

5. Wärmeaustauschvorrichtung (1) nach einem der Ansprüche 2 bis 4, wobei ein Hal-tesack (5) mit Mitteln (54) zum entfernbaren Befestigen an dem äußeren Körper dem Beutel (4, 4') entfernbar zugeordnet ist.

6. Wärmeaustauschvorrichtung (1) nach Anspruch 5, wobei der Haltesack (5) aus einem flexiblen Material besteht.

7. Wärmeaustauschvorrichtung (1) nach Anspruch 5 oder 6, wobei das entfernbare Befestigungsmittel (54) anpassbar ist.

8. Wärmeaustauschvorrichtung (1) nach einem der Ansprüche 5 bis 7, wobei der Hal-tesack (5) eine thermisch isolierende Hauptoberfläche (52) umfasst.

9. Wärmeaustauschvorrichtung (1) nach einem der Ansprüche 5 bis 8, wobei der Hal-tesack (5) eine Hauptoberfläche (51) umfasst, die aus einem antiallergenen Material be-steht.

10. Wärmeaustauschvorrichtung (1) nach einem der vorhergehenden Ansprüche, wobei der wenigstens eine zweite Wärmetauscher (4, 4') mit dem Hauptkörper (2) durch flexible rohrförmige Kanäle (3, 3') verbunden ist.

11. Wärmeaustauschvorrichtung (1) nach Anspruch 10, wobei die flexiblen rohrförmigen Kanäle (3, 3') thermisch isoliert sind.

12. Wärmeaustauschvorrichtung (1) nach Anspruch 10, wobei die flexiblen rohrförmigen Kanäle (3, 3') entfernbar mit dem Hauptkörper (2) und/oder mit dem wenigstens einen zweiten Wärmetauscher (4, 4') verbunden sind.

13. Wärmeaustauschvorrichtung (1) nach einem der vorhergehenden Ansprüche, wobei die primäre Schaltung (6) des Weiteren Mittel (9) zum Umstellen zwischen einem ersten Betriebsmodus zum Erzeugen von Kälte an dem äußeren Körper und einem zweiten Betriebsmodus zum Erzeugen von Wärme an dem äußeren Körper umfasst.

14. Wärmeaustauschvorrichtung (1) nach Anspruch 13, wobei das Umstellmittel ein Vierwegeventil (9) umfasst.

15. Wärmeaustauschvorrichtung (1) nach einem der vorhergehenden Ansprüche, wobei das Mittel (10) zum Bewirken einer Temperaturänderung an dem primären Wärmeträgerfluid einen Luftwärmetauscher (10) umfasst.

16. Wärmeaustauschvorrichtung (1) nach einem der vorhergehenden Ansprüche, wobei die sekundäre Schaltung (7) des Weiteren ein selektiv betätigbares Elektroheizelement (12) umfasst.

17. Wärmeaustauschvorrichtung (1) nach einem der vorhergehenden Ansprüche, wobei das Expansionsglied (13) der primären Schaltung unter einem Kapillar- und einem Thermostatventil ausgewählt ist.

18. Wärmeaustauschvorrichtung (1) nach einem der vorhergehenden Ansprüche, wobei das primäre Wärmeträgerfluid und das sekundäre Wärmeträgerfluid ein Kühlmittel beziehungsweise Wasser sind.

19. Wärmetauschvorrichtung (1) nach einem der vorhergehenden Ansprüche, wobei die sekundäre Schaltung (7) eine Pumpe (14) zum erzwungenen Umwälzen des zweiten Wärmeträgerfluides umfasst.

20. Wärmeaustauschvorrichtung (1) nach einem der vorhergehenden Ansprüche, des Weiteren umfassend Mittel (24-26, 34) zum Steuern bzw. Regeln und Anpassen des Wärmeaustausches zwischen dem sekundären Wärmeträgerfluid und dem äußeren Körper.

21. Wärmeaustauschvorrichtung (1) nach Anspruch 20, wobei das Steuer- bzw. Regel- und Anpassmittel (24-26, 34) einen Zeitgeber (26), einen Modusauswähler (34) und/oder eine programmierbare elektronische Steuer- bzw. Regeleinheit (24) umfasst.

22. Wärmeaustauschvorrichtung (1) nach einem der vorhergehenden Ansprüche, die mit Wechselstrom betrieben wird.

23. Wärmeaustauschvorrichtung nach einem der vorhergehenden Ansprüche, die eine Energieversorgungsbatterie umfasst.

24. Wärmeaustauschvorrichtung (1) nach einem der vorhergehenden Ansprüche, wobei der Hauptkörper (2) tragbar ist.

## Revendications

1. Appareil d'échange de chaleur (1) comprenant :
- un circuit primaire (6) destiné à faire circuler un fluide porteur thermique primaire, ledit circuit primaire étant enfermé dans un corps principal (2) et comprenant au moins un compresseur (8), des moyens (10) destinés à imposer une variation de température audit fluide porteur thermique primaire, un élément d'expansion (13) dudit fluide porteur thermique primaire et un premier échangeur de chaleur (11), au niveau duquel ledit fluide porteur thermique primaire subit une variation de température de signe opposé ; et
- un circuit secondaire fermé (7) destiné à faire circuler un fluide porteur thermique secondaire, qui est couplé de manière ther-mique avec ledit circuit primaire au niveau dudit premier échangeur de chaleur (11), ledit circuit secondaire comprenant :
a) au moins un second échangeur de chaleur (4, 4') entre ledit fluide porteur thermique secondaire et un corps extérieur, ledit second échangeur de chaleur (4, 4') se situant à l'extérieur dudit corps principal (2) ; et
b) un réservoir de stockage (18) du fluide porteur thermique secondaire ;
**caractérisé en ce que** ledit circuit secondaire (7) comprend en outre une vanne de dérivation (35) qui est adaptée, dans le cas d'une détection de surpression dans le circuit secondaire, de façon à per-mettre l'envoi d'une partie du débit du porteur thermique secondaire vers le réservoir de stockage (18) sans qu'il circule à travers le second échangeur de chaleur (4, 4').

2. Appareil d'échange de chaleur (1) selon la revendication 1, dans lequel ledit au moins un second échangeur de chaleur comprend un sac (4, 4') réalisé dans un matériau flexible, ledit sac pouvant être véhi-culé (41) par ledit fluide porteur thermique secondaire.

3. Appareil d' échange de chaleur (1) selon la revendication 2, dans lequel ledit matériau flexible est du polychlorure de vinyle.

4. Appareil d'échange de chaleur (1) selon la revendication 2 ou la revendication 3, dans lequel ledit sac (4, 4') comprend un chemin en zigzag (41) entre des parties thermoscellées (42) des parois dudit sac (4, 4').

5. Appareil d'échange de chaleur (1) selon l'une quelconque des revendications 2 à 4, dans lequel un sac de support (5) qui présente des moyens (54) destinés à le fixer de manière amovible sur ledit corps extérieur, est associé de manière amovible audit sac (4, 4').

6. Appareil d'échange de chaleur (1) selon la revendication 5, dans lequel ledit sac de support (5) est réalisé dans un matériau flexible.

7. Appareil d'échange de chaleur (1) selon la revendication 5 ou la revendication 6, dans lequel lesdits moyens de fixation amovibles (54) sont réglables.

8. Appareil d'échange de chaleur (1) selon l'une quelconque des revendications 5 à 7, dans lequel ledit sac de support (5) comprend une surface principale (52) isolante de manière thermique.

9. Appareil d'échange de chaleur (1) selon l'une quelconque des revendications 5 à 8, dans lequel ledit sac de support (5) comprend une surface principale (51) réalisée dans un matériau antiallergique.

10. Appareil d'échange de chaleur (1) selon l'une quelconque des revendications précédentes, dans lequel ledit ou lesdits seconds échangeurs de chaleur (4, 4') sont connectés audit corps principal (2) par l'intermédiaire de conduits tubulaires flexibles (3, 3').

11. Appareil d'échange de chaleur (1) selon la revendication 10, dans lequel lesdits conduits tubulaires flexibles (3, 3') sont isolés de manière thermique.

12. Appareil d'échange de chaleur (1) selon la revendication 10, dans lequel lesdits conduits tubulaires flexibles (3, 3') sont connectés de manière amovible audit corps principal (2) et/ou audit au moins un second échangeur de chaleur (4, 4').

13. Appareil d'échange de chaleur (1) selon l'une quelconque des revendications précédentes, dans lequel ledit circuit primaire (6) comprend en outre des moyens (9) destinés à commuter entre un premier mode de fonctionnement destiné à produire du froid au niveau dudit corps extérieur et un second mode de fonctionnement destiné à produire de la chaleur au niveau dudit corps extérieur.

14. Appareil d'échange de chaleur (1) selon la revendication 13, dans lequel lesdits moyens de commutation comprennent une vanne à quatre voies (9).

15. Appareil d'échange de chaleur (1) selon l'une quelconque des revendications précédentes, dans lequel lesdits moyens (10) destinés à imposer une variation de température audit fluide porteur thermique primaire comprennent un échangeur de chaleur à air (10).

16. Appareil d'échange de chaleur (1) selon l'une quelconque des revendications précédentes, dans lequel ledit circuit secondaire (7) comprend en outre un élément électrothermique (12) pouvant être actionné de manière sélective.

17. Appareil d'échange de chaleur (1) selon l'une quelconque des revendications précédentes, dans lequel ledit élément d'expansion (13) dudit circuit primaire est sélectionné dans le groupe constitué par un tube capillaire et une vanne thermostatique.

18. Appareil d'échange de chaleur (1) selon l'une quelconque des revendications précédentes, dans lequel ledit fluide porteur thermique primaire et ledit fluide porteur thermique secondaire sont respective-ment un fluide de refroidissement et de l'eau.

19. Appareil d'échange de chaleur (1) selon l'une quelconque des revendications précédentes, dans lequel ledit circuit secondaire (7) comprend une pompe (14) destinée à faire circuler de force ledit fluide porteur thermique secondaire.

20. Appareil d'échange de chaleur (1) selon l'une quelconque des revendications précédentes, comprenant en outre des moyens (24 - 26, 34) destinés à commander et à régler l'échange de chaleur entre ledit fluide porteur thermique secondaire et ledit corps extérieur.

21. Appareil d'échange de chaleur (1) selon la revendication 20, dans lequel lesdits moyens de commande et de réglage (24 - 26, 34) comprennent une minuterie (26), un dispositif de sélection de mode (34) et/ou un boîtier de commande électronique programmable (24).

22. Appareil d'échange de chaleur (1) selon l'une quelconque des revendications précédentes, alimenté en courant alternatif.

23. Appareil d'échange de chaleur (1) selon l'une quelconque des revendications précédentes, comprenant une batterie d'alimentation électrique.

24. Appareil d'échange de chaleur (1) selon l'une quelconque des revendications précédentes, dans lequel ledit corps principal (2) est portable.
